# EUROPÄISCHE PATENTSCHRIFT

(11) **EP 0 388 964 B1**
(45) Veröffentlichungstag und Bekanntmachung des Hinweises auf die Patenterteilung: **02.11.1994**
(21) Anmeldenummer: 90105448.6
(22) Anmeldetag: 22.03.1990
(51) Int. Cl.: C12P 21/08, C12N 15/13

(54) **Verfahren zur Herstellung hetero-bispezifischer Antikörper**
Process for the preparation of a hetero-bispecific antibody
Procédé pour la préparation d'un anticorps hétérobispécifique

(30) Priorität: 23.03.1989 DE 3909708
(43) Veröffentlichungstag der Anmeldung: 26.09.1990
(73) Patentinhaber: BOEHRINGER MANNHEIM GMBH, 68298 Mannheim (DE)
(72) Erfinder: Lenz, Helmut, Dr.rer.nat., D-8132 Tutzing (DE); Weidle, Ulrich, Dr.rer.nat., D-8000 München 2 (DE)
(74) Vertreter: Huber, Bernhard, Dipl.-Chem.

(56) Entgegenhaltungen:
- PROCEEDINGS OF THE NATL. ACADEMY OF SCIENCES USA, Band 83, Nr. 5, März 1986, Baltimore, MD (US); U.D. STAERZ et al., Seiten 1453-1457#
- GENE, Band 51, Nr. 1, 1987, Amsterdam (NL); P. BUCKEL et al., Seiten 13-19#
- PROCEEDINGS OF THE NATL. ACADEMY OF SCIENCES USA, Band 83, Nr. 12, Juni 1986, Baltimore, MD (US); G. JUNG et al., Seiten 4479-4483#
- EUROPEAN JOURNAL OF IMMUNOLOGY, Band 17, April 1987, Weinheim (DE); U.D. STAERZ et al., Seiten 571-574#

## Beschreibung

Die Erfindung betrifft ein Verfahren zur Herstellung hetero-bispezifischer Antikörper.

Hetero-bispezifische oder auch heterobifunktionelle Antikörper sind Antikörper, die zwei verschiedene Antigen-Bindungsstellen aufweisen, d.h. gegen zwei verschiedene Epitope gerichtet sind. Heterobifunktionelle Antikörper könnten zur Therapie von Krankheiten eingesetzt werden. So kann ein bispezifischer Antikörper z.B. eine Antigen-Bindungsstelle für ein Zelloberflächen-Antigen einer Krebszelle und eine Antigen-Bindungsstelle für ein bestimmtes Arzneimittel haben. Auf diese Weise kann dann spezifisch ein Therapeutikum an eine Krebszelle herangeführt werden.
Eine andere Variante ist ein bispezifischer Antikörper, dessen eine Antigen-Bindungsstelle gegen ein T-Zell-Oberflächen-Antigen gerichtet ist und dessen andere Antigen-Bindungsstelle gegen eine antigene Determinante eines Virus gerichtet ist. Ein derartiger Antikörper würde es ermöglichen, gezielt Viren im Blut zu zerstören.

Verfahren zur Herstellung von bispezifischen Antikörpern sind bereits bekannt. So wird in Proc. Natl. Acad. Sci., USA, 83 (1986), 4479-4483 und in Eur. J. Immunol., 17 (1987), 571-574 ein Verfahren beschrieben, bei dem zwei verschiedene Antikörper chemisch miteinander verbunden werden unter Verwendung eines Spacers. Dieses Verfahren ist jedoch aufwendig und führt zu einer neuen topologischen Konstellation der Antikörper.

Weiterhin ist aus Proc. Natl. Acad. Sci., USA, 83 (1986), 1453-1457 ein Verfahren bekannt, bei dem zwei Zellinien, die jeweils verschiedene Antikörper sezernieren, miteinander fusioniert werden. Dabei entstehen Hybridomazellen, die den doppelten Chromosomensatz enthalten und die gewünschten heterobifunktionellen Antikörper produzieren. Nachteil dieses Verfahrens ist es, daß die erhaltenen Zellinien häufig nicht stabil sind und für eine Produktion von heterobifunktionellen Antikörpern in industriellem Maßstab daher nicht geeignet sind.

Es war daher Aufgabe der vorliegenden Erfindung, ein Verfahren zur Verfügung zu stellen, mit dem bispezifische Antikörper in guter Ausbeute gewonnen werden können und das in industriellem Maßstab durchgeführt werden kann.

Diese Aufgabe wird gelöst durch ein Verfahren zur Herstellung von bispezifischen monoklonalen Antikörpern, das dadurch gekennzeichnet ist, daß man aus einer Hybridoma-Zellinie, die einen Antikörper mit einer gewünschten Spezifität sezerniert, mindestens die Gene für die leichte Kette, den variablen Teil der schweren Kette sowie die C_{H}1 Domäne isoliert isoliert, in einen eukaryotischen Plasmid-Vektor insertiert, diesen Expressionsvektor in eine Hybridoma-Zellinie, die Antikörper mit einer zweiten gewünschten Spezifität sezerniert, transfiziert, die Zellinie züchtet, die Antikörper gewinnt und den bispezifischen Antikörper abtrennt.

Überraschenderweise gelingt es mit dem erfindungsgemäßen Verfahren, bispezifische Antikörper in hoher Ausbeute zu erhalten. Die erfindungsgemäß verwendeten transfizierten Hybridoma-Zellinien exprimieren sowohl homobifunktionelle Antikörper, die von dem Genom der Hybridoma-Zellinie sowie dem Expressionsvektor kodiert werden als auch einen aus den Genen beider Antikörper gebildeten heterobifunktionellen Antikörper, der eine Antigenbindungsstelle der Hybridoma-Wirtszelle und eine Antigenbindungsstelle des transfizierten Vektors trägt. Überraschenderweise ist die Ausbeute an diesem heterobifunktionellen Antikörper sehr hoch. Der heterobifunktionelle Antikörper kann neben den beiden homofunktionellen Antikörpern in einfacher Weise bestimmt werden, z.B. durch Bindung an eines seiner Antigene, das an einem festen Träger fixiert ist und Bindung an das andere Antigen, welches markiert und löslich eingesetzt wird.

Zur erfindungsgemäßen Herstellung der bispezifischen Antikörper wird ein eukaryotischer Expressionsvektor in eine Hybridoma-Zellinie eingebracht. Der eukaryotische Expressionsvektor enthält als wesentliche Elemente einen selektionierbaren Marker, einen starken Promotor und die Gene für die Expression eines vollständigen oder unvollständigen Antikörpers. Zur Insertion von Immunglobulingenen sind viele verschiedene eukaryotische Plasmidvektoren geeignet, die dem Fachmann bekannt sind. Geeignet sind z.B. die folgenden Vektoren:
a) Vektoren, welche die Phosphotransferase neo exprimieren, wobei die Selektion von Transformanten mit dem Antibiotikum G418 erfolgt. Diese Vektoren sind bei P. Southern and P. Berg, J. Mol. Appl. Genet. 1 (1982), 327-341, beschrieben.
b) Vektoren, welche die E. coli Xanthin-Guanin-Phosphoribosyl-Transferase exprimieren, wobei die Selektion von Transformanten auf Mycophenolsäure-Resistenz erfolgt. Diese Vektoren sind bei R. Mulligan and P. Berg, Proc. Natl. Acad. Sci. USA, 78 (1981), 2072-2076 beschrieben.
c) Vektoren, die "multi-drug resistance"-Gene exprimieren. Die Selektion erfolgt dabei mit Drogen wie z.B. Colchicin, Vinblastin, Adriamycin oder Actinomycin D. Diese Vektoren sind bei S.E. Kane, B.R. Troen, S. Gal, K. Keda, I. Pastan und M.M. Gottesman, Mol. and Cell. Biol. 8 (1988), 3316-3321 sowie bei D.W. Shou, A. Fojo, I.B. Roninson, J.E. Chin, R. Soffir, I. Pastan und M.M. Gottesman, Mol. and Cell. Biol. 6 (1986), 4039-4044 beschrieben.
d) Vektoren, die Dihydrofolat-Reduktase oder eine Mutante mit reduzierter Affinität zu Methotrexat exprimieren. Die Selektion der Transformanten erfolgt durch Wachstum in Methotrexat-haltigen Medien. Die Vektoren sind bei R. Kaufman und P.A. Sharp, J. Mol. Biol. 159 (1982), 601-621 und bei C.C. Simonson et al., Proc. Natl. Acad. Sci., USA 80, (1983), 2495-2499 beschrieben.

Diese Vektoren weisen alle bereits einen selektionierbaren Marker auf. Als Ausgangsvektor kann z.B. das Plasmid pMT010/A⁺ verwendet werden.

In den Vektor werden Immunglobulingene, die mindestens die leichte Kette, den variablen Teil der schweren Kette und die c_{H}1-Domäne eines Antikörpers einer gewünschten Spezifität oder einen vollständigen Antikörper kodieren, in an sich bekannter Weise insertiert. Dabei kann entweder aus einem, den Antikörper einer gewünschten Spezifität produzierenden Hybridoma die entsprechende DNA gewonnen werden und in den Vektor einligiert werden oder es kann die genomische DNA des gewünschten Immunglobulingens verwendet werden (vgl. Buckel et al., Gene 51 (1980) 13-19).

Als Immunglobulingene werden mindestens das Gen für die leichte Kette, den variablen Teil der schweren Kette und die c_{H}1-Domäne des gewünschten Antikörpers verwendet.

Das Gen für die leichte Kette kann das Gen für eine κ- oder für eine λ-Kette sein. Es wird jeweils das vollständige Gen verwendet. Abhängig von der Art des gewünschten Antikörpers wird das Gen für die schwere Kette ausgesucht. Je nach der Klasse des Antikörpers wird das Gen aus der µ, γ1, γ2, γ3, γ4, α1, α2, δ oder ε-Kette ausgewählt. Dabei kann entweder die gesamte Information für die schwere Kette verwendet werden oder nur ein Teil der für die schwere Kette kodierenden DNA eingesetzt werden. Wenn es erforderlich ist, werden die c_{H}2-, c_{H}3- sowie gegebenenfalls die c_{H}4-Domäne abhängig von der gewünschten Klasse des herzustellenden Antikörpers sowie abhängig von der eingesetzten Hybridoma-Zelllinie ausgewählt. Falls die erfindungsgemäß hergestellten bispezifischen Antikörper zur therapeutischen Behandlung am menschlichen Körper bestimmt sind, ist es bevorzugt, die DNA der c_{H}1-, c_{H}2-, c_{H}3- und gegebenenfalls c_{H}4-Domäne von menschlichen Immunglobulinen zu verwenden.

In einer Ausführungsform des erfindungsgemäßen Verfahrens wird ein Expressionsvektor verwendet, der ein κ-Gen für die leichte Kette und ein γ₁-Gen eines Maus- oder humanen Antikörpers enthält.

Ein bevorzugter Bestandteil des eukaryotischen Expressionsvektors ist ein starker Promotor. Promotoren, die eine hohe Produktionsrate erzeugen, sind dem Fachmann bekannt. Geeignet sind beispielsweise der frühe oder späte Promotor von SV40, Immunglobulin-Promotoren für die leichten und schweren Ketten, der Cytomegalie-Virus-Promotor sowie der Metallothionein-Promotor. Ebenso geeignet sind andere konstitutive virale oder zelluläre Promotoren.

Der so zusammengesetzte eukaryotische Expressionsvektor wird in eine Hybridoma-Zellinie transfiziert, die Antikörper mit Antigen-Bindungsstellen A sezerniert. Hierzu sind Hybridoma-Zellinien geeignet, die Antikörper mit der gewünschten Bindungsstelle produzieren. Bevorzugt wird eine Hybridoma-Zellinie eingesetzt, die Antikörper der Klasse erzeugt, aus der die herzustellenden Antikörper stammen. Geeignet sind insbesondere Maus- oder menschliche Hybridoma-Zellinien.

Die Transfektion des Expressionsvektors in die Hybridoma-Zellinie erfolgt in an sich bekannter Weise. Bevorzugt wird die Transfektion durch Elektroporation durchgeführt (Nucleic Acids Res. 15 (1987) 1311-1326; Bio Techniques 6 (1988) 742-751).

Die so gewonnenen transfizierten Zellinien werden auf den entsprechenden Marker hin selektioniert, gezüchtet und die Antikörper dann nach bekannten Methoden gewonnen (z.B. A. Y. Liu, et al., Proc. Natl. Acad. Sci. USA 84 (1987) 3439; S. L. Morrison et al., Proc. Natl. Acad. Sci. USA 81 (1984) 6851).

Die transfizierten Zellinien produzieren sowohl den homobifunktionellen Antikörper, den die Wirts-Hybridoma-Zellinie sekretiert, als auch den homobifunktionellen Antikörper, den der eukaryotische Expressionsvektor kodiert sowie einen heterobifunktionellen Antikörper, der zwei verschiedene Antigen-Bindungsstellen aufweist. Enthält der transfizierte Expressionsvektor nur die Information für die variablen Bereiche eines Antikörpers mit Antigen-Bindungsstelle B, so werden als Antikörperhälften mit Spezifität B solche gebildet, deren konstanter Teil dem der von der Wirtszelle kodierten Antikörper entspricht, so daß bei der Zusammensetzung zweier Antikörperhälften solche bispezifischen Antikörper entstehen, die Antigen-Bindungsstellen A und B und den konstanten Teil der von der Wirts-Hybridoma-Zelle gebildeten Antikörper aufweisen.

Diese Interpretation der Bildung der heterobifunktionellen Antikörper schließt jedoch andere mögliche Bildungsweisen nicht aus und stellt nur einen Erklärungsversuch dar.

Der heterobifunktionelle Antikörper wird in überraschend hoher Ausbeute sezerniert. Es wurde gefunden, daß die Bildung des gewünschten Antikörpers um den Faktor 10 erhöht ist gegenüber Hybridoma-Wirtszellen, die selbst kein Immunglobulin produzieren können und mit Expressionskonstrukten für leichte und schwere Ketten von monoklonalen Antikörpern transfektiert sind. Das bedeutet, daß die erfindungsgemäß hergestellten Zellinien eine um den Faktor 10 erhöhte Rate der Antikörper-Produktion zeigen.

Aus dem Kulturüberstand, der eine Mischung von zehn verschiedenen Antikörperarten (alle möglichen Kombinationen der leichten und schweren Ketten beider Antikörper) enthält, kann der gesuchte Antikörper nach bekannten Methoden abgetrennt werden. Bevorzugt wird der gewünschte Antikörper durch Immunsorption abgetrennt. In einer besonders bevorzugten Ausführungsform erfolgt die Abtrennung des bifunktionellen Antikörpers in zwei Stufen. Zuerst wird die alle Antikörper-Arten enthaltende Lösung mit einem mit der Antigen-Bindungsstelle A bindefähigen Antigen in Verbindung gebracht, wobei die Antikörper A und AB adsorbiert werden. Nach Elution der gebundenen Antikörper wird in einem zweiten Schritt aus der homo- und bifunktionelle Antikörper enthaltenden Lösung durch Adsorption an ein mit der Antigen-Bindungsstelle B bindefähigen Antigen der heterobifunktionelle Antikörper selektiv von dem homobifunktionellen Antikörper A abgetrennt. Durch Elution können die hetero-bifunktionellen Antikörper in hoher Reinheit erhalten werden.

Erfindungsgemäß wird ein Verfahren zur Verfügung gestellt, mit dem sich in ausgezeichneter Ausbeute heterobifunktionelle Antikörper gewinnen lassen. Diese Antikörper eignen sich vor allem zur Verwendung in der Therapie von Krebs oder durch Viren hervorgerufene Krankheiten. Je nach Verwendungszweck können die antigenen Determinanten, die gewünscht sind, ausgewählt werden. Es bleibt daher dem Therapeuten ein weites Feld, um die jeweils günstigste Kombination zweiter antigener Determinanten zu finden. Darüberhinaus besteht noch die Möglichkeit, den Fc-Teil des Antikörpers, der für verschiedene biologische Vorgänge verantwortlich ist, zu variieren.

Die Erfindung wird durch die folgenden Figuren und Beispiele erläutert:
- Figur 1: zeigt die Vorkonstruktion für die Expressionsplasmide
- Figur 2: zeigt die verwendeten Expressions-Vektoren
Die Plasmide pBMS1 (DSM 5229) und pBMS2 (DSM 5230) sowie der Mikroorganismus E. coli HB101 (DSM 1607) sind bei der Deutschen Sammlung von Mikroorganismen hinterlegt. Die Hybridom-Zellinie MAK33 ist unter der Nummer ECACC 88091404 bei der European Collection of Animal Cell Cultures hinterlegt.

### Beispiel 1

### Isolierung von Immunglobulingenen

Es wurden nach dem in F. Sablitzky, G. Wildner und K. Rajewsky, EMBO J. 4 (1985) 345-350 und Chr. Kocks und K. Rajewsky, Proc. Natl. Acad. Sci. USA (1988) 85, 8206-8210 beschriebenen Verfahren aus der Maus-Hybridom-Linie A20/44, die anti-idiotypischen Antikörper (Antikörper B), die gegen monoklonale Maus λ, γ₂ₐ Anti-Nitrophenol-Antikörper gerichtet sind, sezerniert, die κ und γ₁-Gene des monoklonalen Antikörpers isoliert.

Die aktiven Gene wurden mit SalI-Linkern (CGTCGACG) versehen und in pUC18 oder pBR322 subkloniert. Das Gen für die leichte Kette wurden von einem 5,5 kb Fragment, das Gen für die schwere Kette von einem 9,25 kb Fragment codiert.

### Beispiel 2

### Konstruktion eines Expressions-Vektors für Immunglobulin-Gene

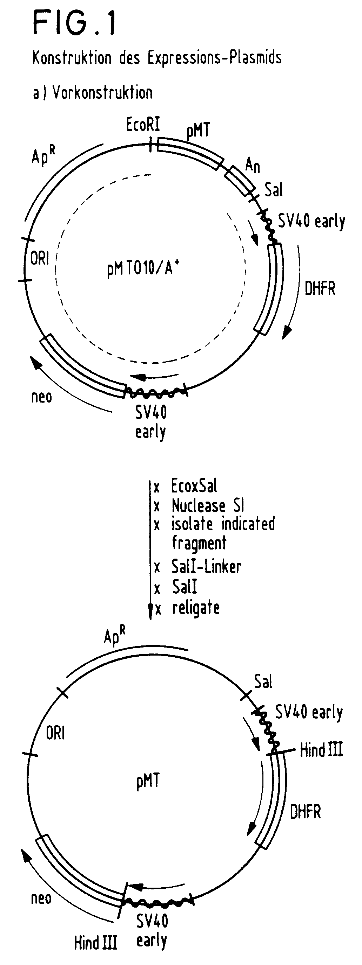

Als Ausgangsplasmid wurde pMTO10/A⁺ verwendet (DNA 5 (1986) 529-537). Dieses Plasmid enthält einen Schaf-Metallothionein Promotor (pMT) mit nachgeschalteter Polyadenylierungs-Stelle (Aₙ), eine Expressions-Kassette für das Maus-Dihydrofolat-Reduktase-Gen unter der Kontrolle des frühen Promotors von SV40, eine Expressions-Kassette für die Phosphotransferase neo unter der Kontrolle des frühen Promotors von SV40, einen bakteriellen Replikations-Ursprung und ein Gen, welches für Ampicillin-Resistenz kodiert. Der Schaf-Metallothionein-Promotor mit zugehöriger Polyadenylierungs-Stelle wurde durch Spalten des Plasmids mit EcoRI und SalI herausgeschnitten, die Fragmente mit Nuklease S1 behandelt und das in Fig. 1 durch eine gestrichelte Linie gekennzeichnete Fragment aus einem niedrig-schmelzenden Agarose-Gel isoliert. Anschließend wurden SalI-Linker anligiert, mit SalI nachgeschnitten, das mit Linker versehene Fragment auf einem niedrig-schmelzenden Agarose-Gel isoliert, religiert (T4-Ligase), in E. coli HB101 (DSM 1607) transfiziert und Ampicillin-resistente Kolonien isoliert. Plasmid pMT wurde durch Restriktions-Analyse charakterisiert (Spaltung mit HindIII ergab zwei Fragmente von 2,8 und 4,65 kb). Das an den Enden mit SalI-Schnittstellen versehene κ-Gen aus der Hybridom-Linie A20/44 wurde in die SalI-Stelle von Plasmid pMT einligiert. Das entstandene Plasmid wurde partiell mit SalI gespalten und das γ1-Gen aus der Hybridom-Zelllinie pA20/44 als 9,25 kb Sal-Fragment einligiert.

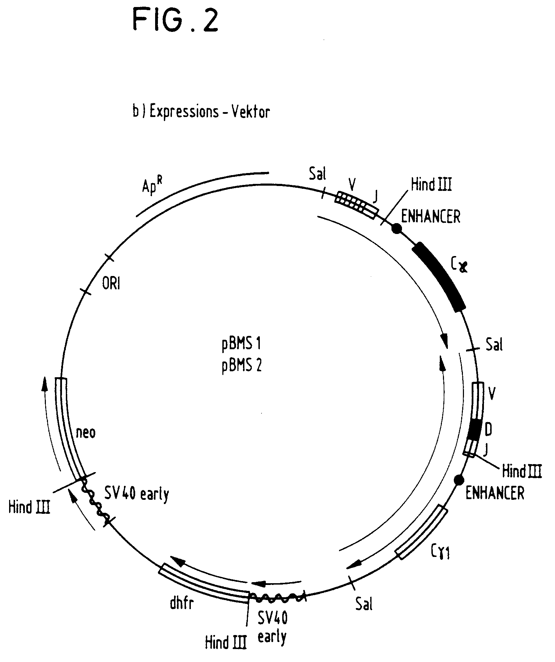

Hierbei entstanden die Plasmide pBMS1 und pBMS2 entsprechend den unterschiedlichen Orientierungen des 1-Gens bezüglich des κ-Gens (Fig. 2). Spaltung von Plasmid pBMS1 mit HindIII ergab Fragmente von 8,6; 6,6; 4,2 und 2,8 kb, Spaltung von Plasmid pBMS2 mit HindIII ergab Fragmente von 11,5; 6,6; 2,8 und 1,3 kb.

### Beispiel 3

### Transfektion einer Hybridoma-produzierenden Zellinie mit Expressionskonstrukten für Immunglobulin-Genen.

Die Plasmide pBMS1 und pBMS2 wurden durch Elektroporation (Gene Pulser von Bio-Rad) in die bei Buckel et al., Gene 51 (1980) 13-19 beschriebene Hybridom-Linie MAK33 (ECACC 88091404), die gegen die Untereinheit M von Creatinkinase gerichtete Antikörper sezerniert (Antikörper A), eingeführt nach dem in Nucleic Acids Res. 15 (1987) 1311-1326 bzw. Bio Techniques 6 (1988) 742-751 angegebenen Verfahren. Pro Puls wurden 10 µg Plasmid-DNA und 1x10⁶ Zellen eingesetzt.

Nach Abzentrifugieren wurden die Zellen mit kaltem HeBS-Puffer gewaschen (20 mM HEPES, pH 7,05, 137 mM NaCl, 5 mM KCl, 0,7 mM Na₂HPO₄, 6 mM Dextrose), mit HeBS-Puffer resuspendiert, auf eine Konzentration von 10⁶ Zellen/ml eingestellt und auf Eis gestellt. Nach Plasmid-Zugabe wurde gepulst (Bedingungen: Kapazität 500 µF und Spannungsbereich zwischen 240 und 350 V oder bei 160 µF und 200 bis 260 V). Die Zellen wurden nach dem Pulsen ca. 10 Minuten auf Eis gehalten und anschließend in Medium I (RPMI 1640, 10 % foetales Kälberserum, 2 mmol/l Glutamin, 1 mmol/l Natriumpyruvat, 0,1 mol/l nicht essentielle Aminosäuren) bei 37°C inkubiert.

Die Selektion stabiler Transformanten erfolgte durch Inkubation auf G418-haltigem Medium bei einer Konzentration von 800 µg G418/ml Medium.

Stabile Transformanten wurden nach etwa 14 Tagen identifiziert und werden durch "limited dilution" isoliert und in Massenkultur in Medium I propagiert.

### Beispiel 4

### Bestimmung von immunreaktivem Protein in den Überständen

Die Bestimmung wurde wie in Gene 56 (1987) 205-216 mit einem ELISA-Test durchgeführt. Zur Bestimmung der Kappa-Ketten wurden Mikrotiterplatten mit Anti-Maus-IgG-Antikörpern aus Schaf beschichtet und mit Überständen aus den transfizierten Zellen inkubiert. Nach Waschen wurde zur Markierung ein Konjugat aus Peroxidase und Fab-Fragmenten eines Schaf-Antikörpers (IgG) der gegen Maus-Fab-Fragmente (IgG) gerichtet war, zugegeben. Anschließend wurde als Substrat für Peroxidase ABTS (2,2-Azino-di-[3-ethyl-benzthiazolin-Sulfonat(6)]) zugegeben und die Farbbildung bei 405 nm als Maß für die Menge an in der Lösung vorhandener Kappa-Kette gemessen.

Die Gamma-Ketten wurden analog bestimmt, wobei jedoch als markierter Rezeptor ein Konjugat aus Peroxidase und Fab-Fragmenten eines Antikörpers aus Schaf (IgG), der gegen Maus Fc-Gamma (IgG) gerichtet war, für die Nachweisreaktion verwendet wurde.

### Beispiel 5

### Auftrennung der Antikörper

Die Kulturüberstände, enthaltend Antikörper mit Spezifität A, B und AB wurden an einer Affinitätssäule chromatographiert, an der mit dem Antikörper A bindefähiges Antigen gebunden war. Antikörper B konnte auf diese Weise abgetrennt werden. Antikörper A und der Hybrid-Antikörper AB wurden an den immobilisierten Antigenen gebunden. Anschließend wurden die Antikörper A und die Hybrid-Antikörper AB mit einer Lösung von 0,2 mol/l Glycin, pH 2,8, eluiert und das Eluat an einer Affinitäts-Säule, an der mit Antikörper B bindefähiges Antigen immobilisiert war, chromatographiert. Der Antikörper A befand sich in der aus der Säule ablaufenden Flüssigkeit, Antikörper AB wurde selektiv gebunden und konnte durch Elution mit einer Lösung von 0,2 mol/l Glycin, pH 2,8, rein erhalten werden.

### Definition der Antikörper

Antikörper A:
CK-MM-spezifischer Antikörper der Hybridoma-Linie MAK 33
Antikörper B:
Antiidiotypischer Antikörper aus Zellinie A 20/44 nach Beispiel 1
Antikörper AB
Hybrid-Antikörper aus Antikörper A und B

### Beispiel 6

### Herstellung von biotinylierter CK-MM

CK-MM (Creatinkinase, EC 2.7.3.2) wurde nach Keutel et al., Arch. Biochem. Biophys. 150 (1972) 648, aus humanem Skelettmuskel isoliert. Zu einer Lösung von 10 mg CK-MM (0,12 µMol) in 30 mM Kaliumphosphatpuffer, pH 7,1 wurden 0,43 µMol Biotinyl-ε-aminocapronsäure-N-hydroxysuccinimidester in 50 µl Dimethylsulfoxid pipettiert und 60 Minuten im Eisbad inkubiert. Anschließend wurde der Überschuß an Reagenz durch Dialyse gegen 2 mM Carbonatpuffer, pH 8,7, entfernt und die biotinylierte CKMM lyophilisiert.

### Beispiel 7

### Spezifische Bestimmung des Hybrid-Antikörpers AB in den Kultur-Überständen

Die Vertiefungen einer Mikrotiterplatte wurden mit Thermo-RSA-Streptavidin (10 µg/ml in 40 mmol/l Kalium-Phosphat, pH 7,4 (Herstellung nach EP-A 0 269 092) beschichtet. Nach Nachbeschichtung mit Puffer I (50 mmol/l HEPES, 0,15 mol/l NaCl, 1 % Crotein C, pH 7,0) wurde mit biotinylierter humaner CK-MM (Herstellung nach Beispiel 6) (1 µg/ml in Puffer I) beladen. Anschließend wurde mit Zellkultur-Überständen, die den Hybrid-MAK AB enthielten, eine Stunde bei Raumtemperatur inkubiert und anschließend zweimal gewaschen mit Inkubations-Puffer (Zusammensetzung: 50 mmol/l HEPES, pH 7,0, 0,15 mol/l NaCl, 1 % Crotein C, 0,2 mol/l Di-Natrium-Tartrat, 0,75 % PEG 40.000, 0,5 % Pluronic® F68, 0,01 % Phenol). Danach wurde mit POD-markierten Fab-Fragmenten (127 mU/ml POD-Aktivität) eines gegen den Antikörper B gerichteten Antikörpers (Antikörper C) inkubiert. Konjugat-Reste wurden durch dreimaliges Waschen mit 50 mmol/l HEPES, pH 7,0, 0,15 mmol/l NaCl, 0,1 % Pluronic® F68, entfernt. Zur Bestimmung der Markierung wurde dann als Substrat für POD ABTS [2,2′-azino-di- 3-ethylbenzthiazolin-sulfonat] zugegeben, eine Stunde inkubiert und dann die Extinktion bei 405 nm in einem Photometer (ELISA-Reader) gemessen. Eine Eichkurve wurde mit einem Modell-Hybrid Antikörper erstellt, der durch Quervernetzung von Antikörper B mit Fab-Fragmenten von Antikörper A erhalten wurde. Die eingesetzten Konzentrationen des Standards lagen bei 0 bis 11,5 ng/ml.

### Beispiel 8

### Bestimmung von Antikörper B

Mikrotiterplatten wurden mit Antikörper C (gerichtet gegen Nitrophenol) beschichtet. Der Beschichtungspuffer bestand aus 0,2 mmol/l Carbonat/Bicarbonat, pH 9,4. Nach 2 Stunden wurden die Platten mit einem Nachbeschichtungspuffer (50 mmol/l HEPES, 0,15 mol/l NaCl, 1 % Crotein C, pH 7,0) 30 Minuten inkubiert. Alle Reaktionen wurden bei Raumtemperatur unter Schütteln durchgeführt. Die Eichkurve wurde mit Antikörper B enthaltenden Standardlösung erstellt. Die Eichproben und die Zellkulturüberstände wurden mit Medium I verdünnt und zwei Stunden bei Raumtemperatur inkubiert. Nach Aussaugen der Vertiefungen und zweimaligem Waschen mit Inkubationspuffer (50 mmol/l HEPES, 0,15 mmol/l NaCl, 0,2 mol/l Di-Natriumtartrat, 1 % Crotein C, 0,75 % PEG 40 000, 0,5 % Pluronic® F68, 0,01 % Phenol, pH 7,0) erfolgte die einstündige Konjugatinkubation. Dazu wurde ein Konjugat aus Fab-Fragmenten von Antikörper C und POD verwendet, das in Inkubationspuffer auf 159 mU/ml POD-Aktivität verdünnt wurde. Nach Aussaugen und dreimaligem Waschen der Vertiefungen mit Waschpuffer (50 mmol/l HEPES, 0,15 mmol/l NaCl, 0,1 % Pluronic® F68, pH 7,0) wurde 60 Minuten mit ABTS als Substrat umgesetzt. Die Extinktion wurde im Photometer (ELISA-Reader) bei 405 nm gegen 490 nm gemessen. Die Konzentrationen der Proben wurden über die Standard-Eichkurve bestimmt.

### Beispiel 9

### Bestimmung von Antikörper A und AB

Thermo-RSA-Streptavidin (10 µg/ml) wurde an eine feste Phase (Mikrotiterplatte mit 96 Vertiefungen, Nunc) adsorbiert in Beschichtungspuffer (40 mmol/l Kaliumphosphatpuffer, pH 7,4). Nicht gebundenes Streptavidin wurde sorgfältig entfernt und unspezifische Bindungsstellen mit Nachbeschichtungs-Lösung (50 mmol/l Kaliumphosphatpuffer, 0,15 mol/l NaCl, 1 % RSA, pH 7,5) abgesättigt. Die Eichproben (Antikörper A = CK-MM spezifisch) und die Kulturüberstände wurden vor dem Auftragen 1:10 mit gemäß Beispiel 6 erhaltener biotinylierter CK-MM (500 ng/ml) verdünnt. Nach einer Stunde Probeninkubation wurde zweimal mit obengenanntem Inkubationspuffer gewaschen. Dann wurde mit POD-markiertem gegen den Fc-Teil von Maus IgG gerichtetem polyklonalem Antikörper, (POD-Aktivität 130 mU/ml) hergestellt in Ziege, eine Stunde inkubiert. Nach Inkubation mit dem Substrat ABTS wurde die Extinktion bei 405 nm im Photometer (ELISA-Reader) gemessen.

Der Gehalt an Antikörper A wurde durch Subtraktion der Konzentrationen von Antikörper (A+AB) und Antikörper (AB) ermittelt.

### Beispiel 10

### Bestimmung von Antikörper B und AB

Mikrotiter-Platten wurden mit gegen den Fcγ Teil von Maus IgG gerichteten Schaf IgG in 0,2 mol/l Bicarbonat/-Carbonat-Puffer (pH 9,5) 10 µg/ml beschichtet. Nicht gebundener Antikörper wurde entfernt und die unspezifischen Bindungsstellen durch Nachbeschichtungslösung (50 mmol/l Kaliumphosphat, 0,15 mol/l NaCl, 1 % RSA (Rinderserumalbumin), pH 7,5) abgesättigt. Alle Reaktionen erfolgten bei Raumtemperatur unter Schütteln. Die Kulturüberstände wurden mit 50 mmol/l Kaliumphosphat, pH 7,5, 0,2 mol/l Natrium-Tartrat, 1 % RSA verdünnt und eine Stunde inkubiert. Nach zweimaligem Waschen mit Inkubationspuffer erfolgte Inkubation mit einem Konjugat aus POD und Fab-Fragmenten des Antikörpers C (POD-Aktivität 130 mU/ml). Nach Waschen wurde mit ABTS inkubiert und nach 60 Minuten die Extinktion im Photometer (ELISA-Reader) bei 405 nm gemessen. Eine Eichkurve wurde mit aufgereinigtem Antikörper B angelegt.

Der Gehalt an Antikörper B wurde durch Subtraktion der Konzentrationen von Antikörper B + AB und Antikörper AB berechnet.

### Beispiel 11

### Vergleich der Ausbeuten an Antikörper A, B und AB

Das Plasmid pBMS1 wurde in die die für CK-MM spezifischen Antikörper A produzierende Zellinie MAK33 transfiziert, G418 resistente Kolonien isoliert und nach Aussaat von 10⁶ Zellen in 24 Stunden Überständen folgende Parameter wie beschrieben bestimmt.
a) Antikörper AB
b) Antikörper B
c) Antikörper A

Die Ergebnisse zeigt Tabelle 1

**Tabelle 1**

| Zellinie | Antikörper A (µg/ml) | Antikörper AB (µg/ml) | Antikörper B (µg/ml) |
|---|---|---|---|
| 1 | 9 | 2,2 | 0,2 |
| 2 | 6,5 | 2,7 | 0,3 |
| 3 | 7 | 2,3 | 0,1 |
| 4 | 4,8 | 2,2 | 0,3 |
| 5 | 7 | 2,5 | 0,2 |
| 6 | 11,8 | 0,7 | 0,05 |
| 7 | 7 | 1,6 | 0,3 |
| 8 | 5 | 1 | 0,1 |
| 9 | 11 | 0,6 | 0,05 |
| 10 | 9 | 1,8 | 0,3 |
| 11 | 5,4 | 3 | 0,3 |
| 12 | 7 | 0,8 | 0,1 |

Ähnliche Ergebnisse wurden bei Verwendung von Plasmid pBMS2 erhalten.

## Patentansprüche

1. Verfahren zur Herstellung von hetero-bispezifischen monoklonalen Antikörpern mit einer Antigenbindungsstelle A und einer Antigenbindungsstelle B, **dadurch gekennzeichnet,** daß man aus einer Hybridoma-Zellinie, die einen Antikörper mit Antigenbindungsstellen A sezerniert, mindestens die Gene für die leichte Kette, für den variablen Teil der schweren Kette sowie die c_{H}1 Domäne isoliert, in einen eukaryotischen Plasmid-Vektor insertiert, diesen Expressionsvektor in eine Hybridoma-Zellinie, die Antikörper mit Antigenbindungsstellen B sezerniert, transfiziert, die Zellinie züchtet, die Antikörper gewinnt und den bispezifischen Antikörper abtrennt.

2. Verfahren nach Anspruch 1, **dadurch gekennzeichnet,** daß man mit einem eukaryotischen Expressionsvektor transfiziert, der die gesamte genetische Information für einen vollständigen Antikörper enthält.

3. Verfahren nach Anspruch 1, **dadurch gekennzeichnet,** daß man einen Expressionsvektor verwendet, der das κ-Gen sowie das Gen für die γ1-Kette eines Maus-Antikörpers enthält.

4. Verfahren nach einem der vorhergehenden Ansprüche, **dadurch gekennzeichnet,** daß man einen Expressionsvektor verwendet, der einen selektionierbaren Marker und einen starken Promotor enthält.

5. Verfahren nach Anspruch 4, **dadurch gekennzeichnet,** daß man als Expressionsvektor das Plasmid pBMS1 (DSM 5229) oder pBMS2 (DSM 5230) verwendet.

6. Verfahren nach einem der vorhergehenden Ansprüche, **dadurch gekennzeichnet,** daß man als Promotor den frühen oder späten Promotor von SV40, den Cytomegalie-Virus-Promotor oder den Metallothionein-Promotor verwendet.

7. Verfahren nach einem der vorhergehenden Ansprüche, **dadurch gekennzeichnet,** daß die Transfektion der Hybridoma-Zellinie mit dem eukaryotischen Expressionsvektor durch Elektroporation erfolgt.

## Claims

1. Process for the production of hetero-bispecific monoclonal antibodies with an antigen binding site A and an antigen binding site B, **wherein** at least the genes for the light chain, for the variable part of the heavy chain, as well as the c_{H}1 domain are isolated from a hybridoma cell line which secretes an antibody with antigen binding sites A and are inserted into a eukaryotic plasmid vector, this expression vector is transfected into a hybridoma cell line which secretes antibodies with antigen binding sites B, the cell line is cultured, the antibodies are obtained and the bispecific antibody is isolated.

2. Process as claimed in claim 1, **wherein** one transfects with a eukaryotic expression vector which contains the entire genetic information for a complete antibody.

3. Process as claimed in claim 1, **wherein** an expression vector is used which contains the κ gene as well as the gene for the γ₁ chain of a mouse antibody.

4. Process as claimed in one of the previous claims, **wherein** an expression vector is used which contains a marker capable of selection and a strong promoter.

5. Process as claimed in claim 4, **wherein** plasmid pBMS1 (DSM 5229) or pBMS2 (DSM 5230) is used as the expression vector.

6. Process as claimed in one of the previous claims, **wherein** the early or late promoter of SV40, the cytomegalo-virus immediate-early promoter or the metallothionein promoter is used as the promoter.

7. Process as claimed in one of the previous claims, **wherein** the transfection of the hybridoma cell line with the eukaryotic expression vector is effected by electroporation.

## Revendications

1. Procédé de préparation d'anticorps monoclonaux hétérobispécifiques ayant un site de liaison d'antigène A et un site de liaison d'antigène B, caractérisé en ce qu'à partir d'une lignée cellulaire d'hybridome qui sécrète un anticorps ayant des sites de liaison d'antigène A on isole au moins les gènes pour la chaîne légère, pour la partie variable de la chaîne lourde et le domaine C_{H}1, on les insère dans un vecteur plasmidique eucaryotique, on transfecte avec ce vecteur d'expression une lignée cellulaire d'hybridome qui sécrète des anticorps ayant des sites de liaison d'antigène B, on cultive la lignée cellulaire, on extrait les anticorps et on sépare l'anticorps bispécifique.

2. Procédé selon la revendication 1, caractérisé en ce que l'on transfecte avec un vecteur d'expression eucaryotique qui contient toute l'information génétique pour un anticorps complet.

3. Procédé selon la revendication 1, caractérisé en ce que l'on utilise un vecteur d'expression qui contient le gêne κ et le gène pour la chaîne γ₁ d'un anticorps de souris.

4. Procédé selon l'une des revendications précédentes, caractérisé en ce que l'on utilise un vecteur d'expression qui contient un marqueur sélectionnable et un promoteur fort.

5. Procédé selon la revendication 4, caractérisé en ce que l'on utilise comme vecteur d'expression le plasmide pBMS1 (DSM 5229) ou pBMS2 (DSM 5230).

6. Procédé selon l'une des revendications précédentes, caractérisé en ce que l'on utilise comme promoteur le promoteur précoce ou tardif de SV40, le promoteur de cytomégalovirus ou le promoteur de la métallothionéine.

7. Procédé selon l'une des revendications précédentes, caractérisé en ce que la transfection de la lignée cellulaire d'hybridome avec le vecteur d'expression eucaryotique est réalisée par électroporation.
